# EUROPEAN PATENT SPECIFICATION

(11) **EP 2 088 185 B1**
(45) Date of publication and mention of the grant of the patent: **02.11.2011**
(21) Application number: 09160925.5
(22) Date of filing: 03.07.2007
(51) Int. Cl.: C10L 1/238, C10L 1/226, C10L 10/18, C10L 10/02, F02M 61/18, F02M 65/00, F02B 77/04, C08F 8/30, C07C 241/04

(54) **Diesel fuel composition**
Dieselkraftstoffzusammensetzung
Composition de carburant diesel

(30) Priority: 04.08.2006 EP 06118488
(43) Date of publication of application: 12.08.2009
(62) Divisional of application: 07111678.4
(73) Proprietor: Infineum International Limited, Abingdon Oxfordshire OX13 6BB (GB)
(72) Inventor: Breakspear, Angella Priscilla, Dr., Abingdon Oxfordshire, OX13 6BB (GB); Caprotti, Rinaldo, Abingdon Oxfordshire, OX13 6BB (GB); Thomson, Russell Martin, Abingdon Oxfordshire, OX13 6BB (GB)
(74) Representative: Capaldi, Michael Joseph

(56) References cited:
- EP-A1- 0 632 123
- WO-A1-02/06428
- AU-B2- 413 800
- GB-A- 1 121 681
- US-A- 3 723 460
- GRAUPNER O ET AL: "INJECTOR DEPOSIT TEST FOR MODERN DIESEL ENGINES", 5TH INTERNATIONAL COLLOQUIUM, 12 January 2005 (2005-01-12), pages 157-162, XP008073687, Technische Akademie Esslingen

## Description

This invention relates to a diesel fuel composition effective for the removal or prevention of fuel injector deposits in diesel engines, in particular to the removal or prevention of fuel injector deposits in modern diesel engines.

There is continued legislative pressure to reduce emissions from diesel engines. In Europe by 2008, all new diesel engines must comply with the Euro V specification. This has resulted in the development of advanced fuel injection equipment characterised by fuel injectors which have complex spray-hole geometries, multiple and narrow spray-holes and which operate with high temperatures and pressures at the injector tips. As a consequence of this increasing severity in operating conditions, the injectors of modern common-rail diesel engines are prone to the formation of deposits. These deposits, which are found both inside and outside the spray-holes of the injector nozzles, contribute directly to loss in engine power and increase in smoke production.

The formation of deposits on diesel fuel injectors is not a new phenomenon and historically any problem has been adequately addressed by the use of conventional diesel detergent additives. It has been observed however, that the types of deposits formed under the more severe operating conditions of engines which are being developed to be Euro V compliant are not adequately removed or prevented by conventional diesel detergent additives. Although not wishing to be bound by any theory, it is presently thought that the formation of injector deposits in modern engines is exacerbated by the presence of minor amounts of metal-containing species in the fuel. Indeed, the Applicant's studies have indicated that the use of fuels with negligible amounts of metal-containing contamination do not result in any significant problems with deposits. However, normal diesel fuels will often contain low but measurable amounts of metal-containing contamination, for example, zinc, copper, iron and lead, and metal-containing species may also be deliberately added to perform other functions. Analysis of the deposits formed in modern diesel engines indicates that, in addition to the expected carbonaceous materials, metals such as zinc and copper can be detected. The present invention specifically addresses the removal and prevention of these new types of injector deposits.

US 3,375,092 discloses the product of the reaction between an alkyl succinic acid or anhydride in which the alkyl radical has from 8 to 24 carbon with a hydrazine. This product is said to be useful as an anti-icing additive for gasoline.

US 2,640,005 discloses succinhydrazides which are formed for example, by the reaction of hydrazine or hydrazine hydrate with the anhydride of a substituted succinic acid. These species are taught as having utility as fungicides.

US 3,723,460 discloses that the species formed by the reaction of e.g. polyisobutenyl-substituted succinic acid or anhydride with hydrazine can be used as fuel and motor oil additives. The intermediate reaction products are preferably post-reacted with further compounds for example, those with displaceable halogens, alkylene oxides etc., but may also be used alone. The species are discussed as being of sufficient detergent strength to clean and maintain clean, a gasoline engine induction system but not of sufficient detergent strength to promote the formation of gasoline-in-water emulsions. They are also able to function as a carburettor cleaner. There is no disclosure of use in diesel engine systems.

WO 2004/029183 discloses ashless anti-wear, anti-fatigue and extreme pressure additives for lubricating oils. These have the formula: 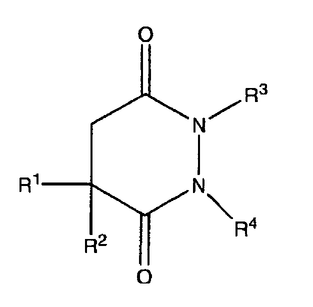 where group R¹ may be e.g. alkyl and groups R²⁻⁴ may be hydrogen or similar to R¹. The additives are prepared by reacting e.g. an alkyl succinic anhydride with hydrazine hydrate.

EP 0 632 123 A1 relates to diesel fuel compositions containing nitrogen-containing dispersants. The dispersant may be chosen from a very wide range of possible species, including those derived from hydrazines. The dispersants are **characterised in that** the numerical value obtained by multiplying the percentage of nitrogen in the dispersant by the weight average molecular weight of the dispersant is between 45,000 and 100,000.

Accordingly, the present invention provides a diesel fuel composition in accordance with claim 1.

It has been found that the reaction products used in the diesel fuel composition of the invention are particularly effective at reducing the incidence of deposits in modern diesel engine fuel injectors, and much more effective than the widely used PIBSA-PAM detergents under similar conditions. It was surprising to note however that in older type diesel engines, such as those used in the industry standard XUD-9 detergency test, the reaction products of use in the present invention were outperformed by conventional PIBSA-PAM detergents.

As discussed above, the incidence of injector deposits appears to be connected to the presence of metal-containing species in the fuel. Some diesel fuels will contain no measurable metal content, in which case the incidence of injector deposits will be reduced. However, the presence or absence of metal-containing species in diesel fuels is generally not apparent to the user and will vary with fuel production, even with fuels from the same supplier. The present invention is thus useful in those instances where metal-containing species are present and also as a preventative measure to lessen the impact of injector deposits when re-fuelling with a fuel of unknown metal content.

In the context of the present invention, substantial removal of injector deposits should be taken to mean that deposits which may be present on the inside or outside of the spray-holes of the injector nozzles are removed to the extent that the proper functioning of the injector is not significantly impaired. This may be determined for example by measuring increases in exhaust smoke or loss in engine torque. It is not required that all traces of injector deposit are removed. Similarly, a reduction in the occurrence of injector deposits does not require that no deposits whatsoever are formed, only again that the amount of any deposit which may form is not sufficient to significantly impair the proper functioning of the injector.

The reaction between the hydrocarbyl-substituted succinic acid or anhydride and hydrazine produces a mixture of reaction products (as discussed hereinbelow). This mixture is made up from species which have a range of molecular weights. These range from low molecular weight species, being composed of one moiety of hydrocarbyl-substituted succinic acid or anhydride and one or two moieties of hydrazine, to species composed of more than one moiety of hydrocarbyl-substituted succinic acid or anhydride and one or more moieties of hydrazine. These latter species have relatively higher molecular weights than the former. It has been observed that most effective detergency is obtained by employing a reaction product which contains a significant proportion of higher molecular weight species.

Expressed in terms of EP 0 632 123 A1 discussed above, preferably, the reaction product used in the present invention is such that the numerical value obtained by multiplying the percentage of nitrogen in the product by the weight average molecular weight of the product is in excess of 105,000, more preferably in excess of 110,000, for example between 110,000 and 250,000.

The various features of the invention, which are applicable to all aspects will now be described in more detail.

### (a) The reaction product

This comprises the product of the reaction between a hydrocarbyl-substituted succinic acid or anhydride and hydrazine.

### (i) Hydrocarbyl-substituted succinic acid or anhydride.

As used in this specification the term "hydrocarbyl" refers to a group having a carbon atom directly attached to the rest of the molecule and having a hydrocarbon or predominantly hydrocarbon character. They may be saturated or unsaturated, linear or branched. Preferably, the hydrocarbyl groups are hydrocarbon groups. These groups may contain non-hydrocarbon substituents provided their presence does not alter the predominantly hydrocarbon character of the group. Examples include keto, halo, nitro, cyano, alkoxy and acyl. The groups may also or alternatively contain atoms other than carbon in a chain otherwise composed of carbon atoms. Suitable hetero atoms include, for example, nitrogen, sulphur, and oxygen. Advantageously, the hydrocarbyl groups are alkyl groups.

Preferably, the hydrocarbyl group of the hydrocarbyl-substituted succinic acid or anhydride comprises a C₈ - C₃₆ group, preferably a C₈ - C₁₈ group. Non-limiting examples include dodecyl, hexadecyl and octadecyl. Alternatively, the hydrocarbyl group may be a polyisobutylene group with a number average molecular weight of between 200 and 2500, preferably between 800 and 1200. Mixtures of species with different length hydrocarbyl groups are also suitable, e.g. a mixture of C₁₆ - C₁₈ groups.

The hydrocarbyl group is attached to a succinic acid or anhydride moiety using methods known in the art. Additionally, or alternatively, suitable hydrocarbyl-substituted succinic acids or anhydrides are commercially available e.g. dodecylsuccinic anhydride (DDSA), hexadecylsuccinic anhydride (HDSA), octadecylsuccinic anhydride (ODSA) and polyisobutylsuccinic anhydride (PIBSA).

### (ii) Hydrazine

Hydrazine has the formula:

NH₂-NH₂

Hydrazine may be hydrated or non-hydrated. Hydrazine monohydrate is preferred.

### (iii) Reaction of (i) and (ii)

The reaction between the hydrocarbyl-substituted succinic acid or anhydride and hydrazine produces a variety of products. As noted above, it is preferable for good detergency that the reaction product contains a significant proportion of species with relatively high molecular weight. The precise nature of the species produced in the reaction has not yet been fully elucidated however, it is presently thought that a major high molecular weight product of the reaction is an oligomeric species predominantly of the structure: 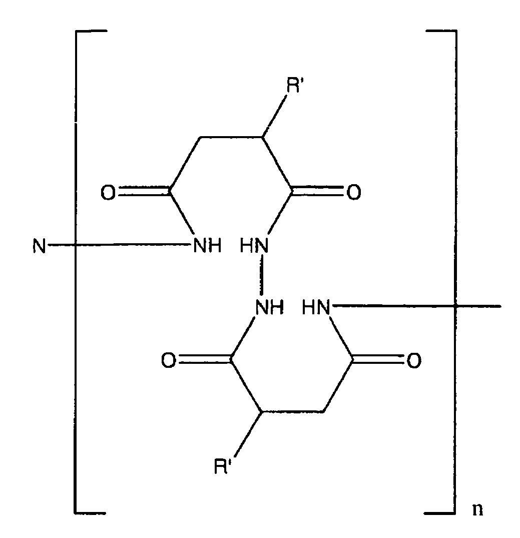 where n is an integer and greater than 1, preferably between 2 and 10, more preferably between 2 and 7, for example 3, 4 or 5. Each end of the oligomer may be capped by one or more of a variety of groups. Some possible examples of these terminal groups include:

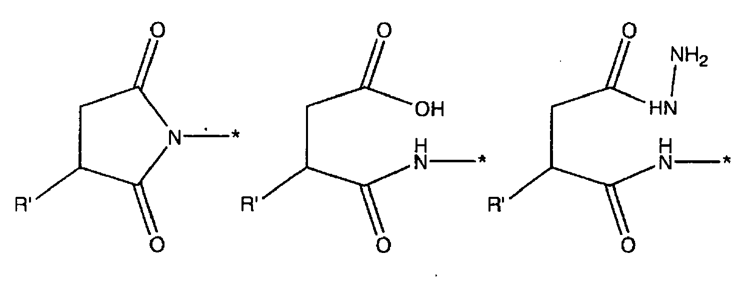

Alternatively, the oligomeric species may form a ring having no terminal groups:

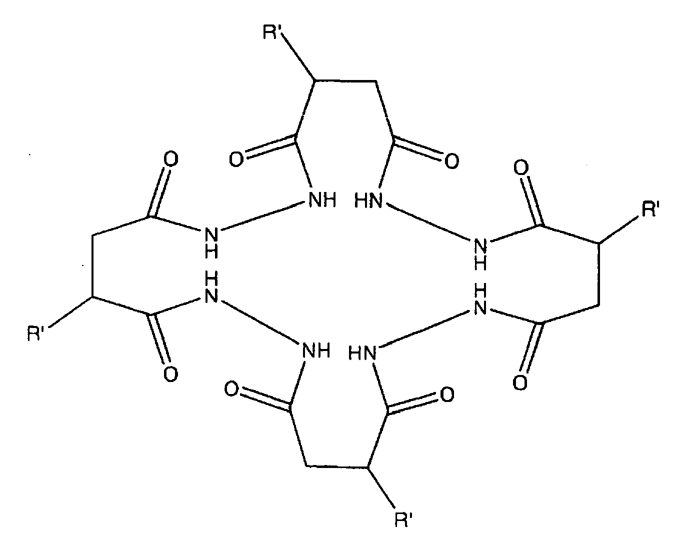

Also thought to be present is a species of the structure: 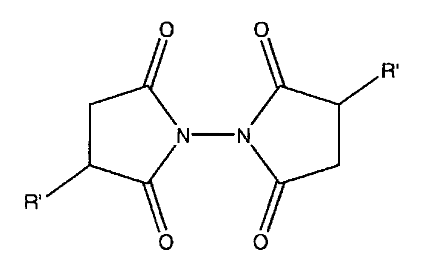 where R' represents the hydrocarbyl substituent. It should be noted that it is also within the scope of the present invention to use more than one hydrocarbyl-substituted succinic acid or anhydride in which case the groups R' in the above structures may be different from one another.

Both of the above structures contain at least two moieties derived from the hydrocarbyl-substituted succinic acid or anhydride. The molecular weights of these species are thus more than twice the average molecular weight of the hydrocarbyl substituent R'. In the context of the present invention the species are thus of relatively high molecular weight.

As lower molecular weight reaction products, species of the following structures are also thought to be present:

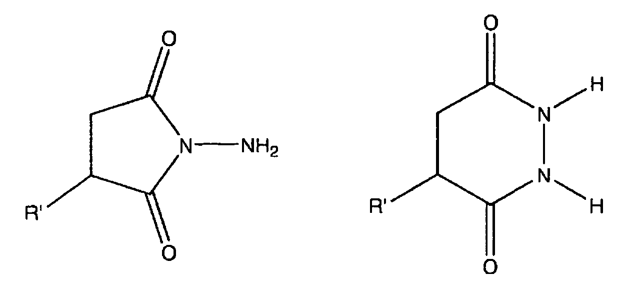

Further possible minor products include:

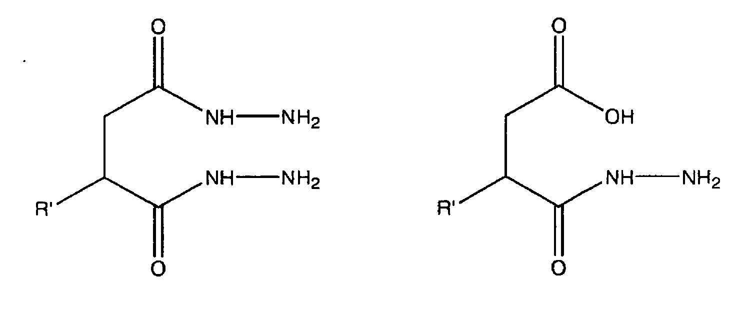

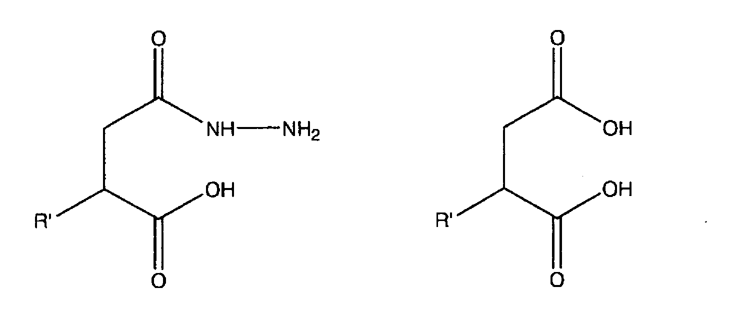

There may also be some salt formation resulting in species of the following structures:

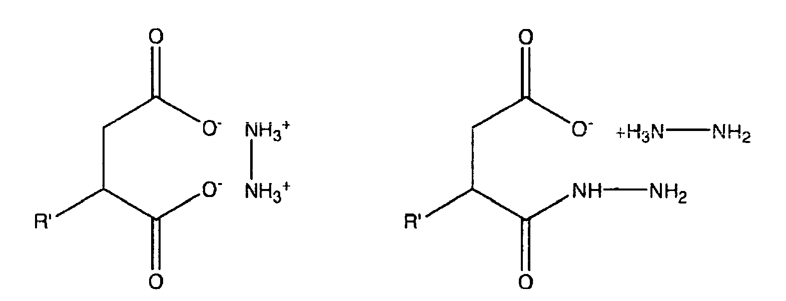

The general synthesis of the reaction products used in the present invention has been described in the art, for example, US 3,375,092, US 2,640,005 and US 3,723,460 cited hereinabove. A range of possible reaction schemes and products has also been given by Feuer et al., in Jn. Amer. Chem. Soc, 73 (1951) pp.4716-4719*.* By way of example a possible preparative route is as follows.

A charge of alkyl-substituted succinic anhydride together with an equal weight of solvent, e.g. toluene is heated to ca. 50°C under nitrogen. The desired amount of hydrazine hydrate is added drop-wise causing an exotherm. Once addition is complete, the reaction mixture is heated to reflux for several hours. The mixture is then water/solvent stripped and the temperature raised to 180°C under reduced pressure.

Preferably, the hydrocarbyl-substituted succinic acid or anhydride and hydrazine are reacted in a molar ratio of between 2:1 and 1:4, more preferably between 1:1-1:3.

Preferably, the reaction product between the hydrocarbyl-substituted succinic acid or anhydride and hydrazine is added to the diesel fuel in an amount of between 20 and 100 ppm by weight, based on the weight of the fuel.

### (b) The diesel fuel

Preferably, the diesel fuel is a petroleum-based fuel oil, especially a middle distillate fuel oil. Such distillate fuel oils generally boil within the range of from 110°C to 500°C, e.g. 150°C to 400°C. The fuel oil may comprise atmospheric distillate or vacuum distillate, cracked gas oil, or a blend in any proportion of straight run and thermally and/or refinery streams such as catalytically cracked and hydro-cracked distillates.

Other examples of diesel fuels include Fischer-Tropsch fuels. Fischer-Tropsch fuels, also known as FT fuels, include those described as gas-to-liquid (GTL) fuels, biomass-to-liquid (BTL) fuels and coal conversion fuels. To make such fuels, syngas (CO + H₂) is first generated and then converted to normal paraffins by a Fischer-Tropsch process. The normal paraffins may then be modified by processes such as catalytic cracking/reforming or isomerisation, hydrocracking and hydroisomerisation to yield a variety of hydrocarbons such as iso-paraffins, cyclo-paraffins and aromatic compounds. The resulting FT fuel can be used as such or in combination with other fuel components and fuel types. Also suitable are diesel fuels derived from plant or animal sources such as FAME. These may be used alone or in combination with other types of fuel.

Preferably, the diesel fuel has a sulphur content of at most 0.05% by weight, more preferably of at most 0.035% by weight, especially of at most 0.015%. Fuels with even lower levels of sulphur are also suitable such as, fuels with less than 50ppm sulphur by weight, preferably less than 20 ppm, for example 10ppm or less.

As discussed herein, the Applicants have observed that the problems associated with the formation of injector deposits in engines being developed to be Euro V compliant are associated with the presence of metal-containing species in the diesel fuel. Commonly when present, metal-containing species will be present as a contaminant, for example through the corrosion of metal and metal oxide surfaces by acidic species present in the fuel. In use, fuels such as diesel fuels routinely come into contact with metal surfaces for example, in vehicle fuelling systems, fuel tanks, fuel transportation means etc. Typically, metal-containing contamination will comprise metals such as zinc, iron, copper and lead.

In addition to metal-containing contamination which may present in diesel fuels there are circumstances where metal-containing species may deliberately be added to the fuel. For example, as is known in the art, metal-containing fuel-borne catalyst species may be added to aid with the regeneration of particulate traps. Such catalysts are often based on metals such as iron, cerium, Group I and Group II metals e.g., calcium and strontium, either as mixtures or alone. Also used are platinum and manganese. The presence of such catalysts may also give rise to injector deposits when the fuels are used in engines being developed to be Euro V compliant.

Metal-containing contamination, depending on its source, may be in the form of insoluble particulates or soluble compounds or complexes. Metal-containing fuel-borne catalysts are often soluble compounds or complexes or colloidal species. It will be understood that metal-containing species in the context of the present invention include both species which are metallic and those where the metal constituent is in compounded form.

In an embodiment, the metal-containing species comprises a fuel-borne catalyst.

In a preferred embodiment, the metal-containing species comprises zinc.

Typically, the amount of metal-containing species in the diesel fuel, expressed in terms of the total weight of metal in the species, is between 0.1 and 50 ppm by weight, for example between 0.1 and 10 ppm by weight, based on the weight of the diesel fuel.

The invention will now be described by way of example only.

### Preparative routes

### Example 1

Dodecylsuccinic anhydride (200g, 0.75 mol) was weighed into a 11, three neck, roundbottom flask together with toluene (200g). Under nitrogen and with stirring, the temperature was raised to ca. 50°C and hydrazine monohydrate (37.59g, 0.75 mol) added dropwise. Once addition was complete, the mixture was heated to reflux for 5 hours. Toluene was removed at 40°C until no more bubbling was seen and then the product was held for 1 hour at 0 mbar and 40°C.

The product produced in the reaction contained around 10% by weight of species having a molecular weight more than 2 times the molecular weight of the hydrocarbyl group of the dodecylsuccinic anhydride reactant.

### Example 2

Dodecylsuccinic anhydride (200g, 0.75 mol) was weighed into a 11, three neck, roundbottom flask together with toluene (200g). Under nitrogen and with stirring, the temperature was raised to ca. 50°C and hydrazine monohydrate (112.76g, 2.25 mol) added dropwise. Once addition was complete, the mixture was heated to reflux for 5 hours. Toluene was removed at 40°C until no more bubbling was seen and then the product was held for 4 hours at 0 mbar and 180°C.

The product produced in the reaction contained around 70% by weight of species having a molecular weight more than 2 times the molecular weight of the hydrocarbyl group of the dodecylsuccinic anhydride reactant.

### Example 3

A further example using dodecylsuccinic anhydride in a synthesis similar to Example 2 produced a product containing around 84% by weight of species having a molecular weight more than 2 times the molecular weight of the hydrocarbyl group of the dodecylsuccinic anhydride reactant.

### Effect of process variables

Example 2 was repeated varying the temperature and pressure of the final stage following the removal of toluene. Table 1 below shows the effect of these variables on the molecular weight distribution of the products obtained. In the Table, high MW species are those having a molecular weight more than 2 times the molecular weight of the hydrocarbyl group of the dodecylsuccinic anhydride reactant.

**Table 1**

| **Temperature / °C** | **Pressure / mbar** | **% of high MW species** |
|---|---|---|
| 40 | 35 | 20 |
| 60 | 35 | 15 |
| 80 | 35 | 17 |
| 100 | 35 | 18 |
| 120 | 35 | 25 |
| 140 | 35 | 33 |
| 160 | 35 | 46 |
| 180 | 35 | 62 |
| 180 | 0 | 76 |

Following the routes of Examples 1 and 2, further species were prepared by reacting hydrazine mono-hydrate with a C₂₄-alkyl succinic anhydride and a polyisobutylene-substituted succinic anhydride (mol weight of PIB ca. 1000).

### Test protocol

The protocol used is described by Graupner et al. "Injector deposit test for modern diesel engines ", Technische Akademie Esslingen, 5th International Colloquium, 12-13 Jan 2005, 3.10, p157, Edited by WilfriedJBartz*.* Briefly, the protocol aims to replicate the operating conditions in a modern diesel engine with an emphasis on the fuel injector tip. The test is split into five stages:
a) an iso-speed measurement of engine power output
b) an 8 hour endurance run
c) an extended soaking period (3 to 8 hours) during which the engine is stopped and allowed to cool
d) a second 8 hour endurance run
e) an iso-speed measurement of engine power output.

For the data presented herein, the five stages above were used however, stages b) ,c) and d) can be repeated any number of times to suit the testing programme being undertaken. Also, stages a) and e) may be omitted but are useful to improve understanding of the results. Results are reported as the difference between the average torque at the start of the test during stage a) and the average torque at the end of the test during stage e). Alternatively, if the isospeed procedure is not run, the measured difference between starting torque at full load/full speed and final load/speed can be used. Differences in smoke production are also noted. The formation of injector deposits will have a negative influence on the final power output and will increase the amount of smoke observed. The injectors used had the physical characteristics (i) - (v) described above.

To replicate the conditions expected in a modern diesel engine, a small amount of metal contamination in the form of zinc neodecanoate was added to the fuel used to run the engine.

The fuel used was a low-sulphur content diesel fuel with the characteristics shown in Table 2 below.

**Table 2**

| **Test description** | **Value** | **Units** |
|---|---|---|
| sulphur content | 0.0005 | mass % |
| cetane number | 55.4 | - |
| density @ 15°C | 844.9 | kgm⁻³ |
| distillation characteristics | | |
| D5% | 204.8 | °C |
| D10% | 211.b | °C |
| D20% | 222.2 | °C |
| D30% | 232.2 | °C |
| D40% | 242.1 | °C |
| D50% | 252.3 | °C |
| D60% | 262.8 | °C |
| D70% | 275.1 | °C |
| D80% | 290.5 | °C |
| D90% | 315.1 | °C |
| D95% | 337.1 | °C |
| FBP | 353.6 | °C |
| IBP | 179.7 | °C |
| kinematic viscosity @ 20°C | 3.935 | cSt |
| kinematic viscosity @ 40°C - D445 | | |
| cloud point | -14.0 | °C |
| CFPP | -33.0 | °C |

For comparative purposes, the species of the invention were tested in the industry standard XUD9 detergency test. A commercial PIBSA-PAM detergent was tested also. The results are given in Table 3 below.

**Table 3**

| **Species** | **Treat rate wppm (active ingredient)** | **Needle lift in XUD9** |
|---|---|---|
| Untreated fuel | - | 92 |
| PIBSA-PAM | 30 | 57 |
| PIBSA-PAM | 60 | 53 |
| PIBSA-PAM | 100 | 8 |
| PIBSA-PAM | 279 | 14 |
| Example 1 | 60 | 83 |
| Example 2 | 60 | 83 |
| Example 3 | 60 | 93 |
| PIB1000 hydrazide | 60 | 77 |
| C₂₄-SA hydrazide | 60 | 78 |
| Example 2 | 300 | 83 |
| PIB1000 hydrazide | 300 | 76 |

These results show that the commercial PIBSA-PAM detergent gave the expected excellent performance in the XUD-9 test. Contrastingly, the hydrazine species performed poorly, even at high treat rates.

The species were then tested using the test protocol described above. Results are given in Table 4 below. 3 ppm of Zn in the form of zinc neodecanoate was added to the fuel for all tests (except for the untreated fuel alone).

**Table 4**

| **Species** | **Treat rate wppm (active ingredient)** | **Torque loss** |
|---|---|---|
| Untreated fuel | - | 4.3% |
| Untreated fuel + 3 ppm Zn | - | 17.2% |
| PIBSA-PAM | 60 | 13.7% |
| PIB1000 hydrazide | 60 | 9.7% |
| C₂₄-SA hydrazide | 60 | 7.1% |
| Example 1 | 60 | 12.0% |
| Example 2 | 60 | 5.2% |

Table 5 below shows a further result for the product of Example 3.

**Table 5**

| **Species** | **Treat rate wppm (active ingredient)** | **Torque loss** |
|---|---|---|
| Untreated fuel | - | 1.3% |
| Untreated fuel + 3 ppm Zn | - | 10.0% |
| PIBSA-PAM | 60 | 9.2% |
| Example 3 | 60 | 0.9% |

The results show that the addition of zinc to the untreated fuel gives rise to a large increase in torque loss. The commercial PIBSA-PAM detergent only gave a marginal improvement. All hydrazine species provided a greater improvement than the commercial detergent. Particularly good performance was obtained for the species of Example 2 and Example 3 which both contained a high proportion of the higher molecular weight species.

The results in Table 6 below also illustrate the effect of the molecular weight distribution of the species on torque loss. Again, all tests contained 3 ppm of Zn in the form of zinc neodecanoate. The species used were the products of the reaction between dodecylsuccinic anhydride and hydrazine mono-hydrate following the routes of Examples 1 and 2 above and were present in the fuel at 60 wppm.

**Table 6**

| **% of high MW species** | **Torque loss** |
|---|---|
| 90.0 | 0.1 % |
| 73.4 | 4.2% |
| 72.0 | 5.2% |
| 70.0 | 5.2% |
| 10.0 | 12.0% |

These results confirm the increased effectiveness of the materials which contain the highest percentage of higher molecular weight species.

## Claims

1. A diesel fuel composition comprising a major amount of a diesel fuel and between 10 and 500 ppm by weight, based on the weight of the fuel of the reaction product between a hydrocarbyl-substituted succinic acid or anhydride and hydrazine, wherein at least 25%, preferably at least 50%, most preferably at least 80% by weight of the reaction product has a molecular weight which is more than 2 times, preferably more than 2.5 times the average molecular weight of the hydrocarbyl group of the hydrocarbyl-substituted succinic acid or anhydride.

2. A diesel fuel composition according to claim 1 wherein the reaction product between the hydrocarbyl-substituted succinic acid or anhydride and hydrazine is predominantly an oligomeric species of the structure: 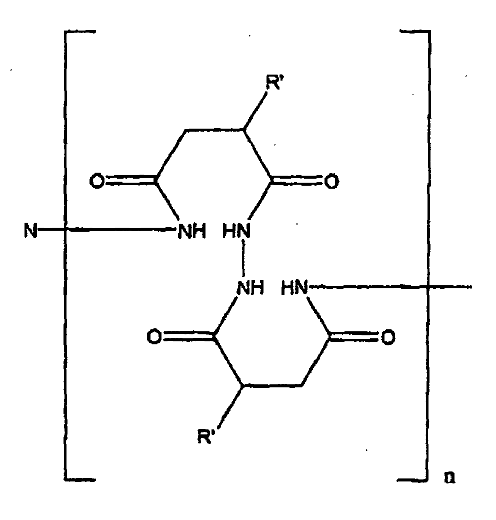 where R' represents the hydrocarbyl substituent and where n is an integer and greater than 1, preferably between 2 and 10, more preferably between 2 and 7, for example 3, 4 or 5.

3. A diesel fuel composition according to claim 1 or claim 2 wherein the hydrocarbyl group of the hydrocarbyl-substituted succinic acid or anhydride comprises a C₈ - C₃₆ group or a polyisobutylene group with a number average molecular weight of between 200 and 2500.

4. A diesel fuel composition according to any preceding claim wherein the hydrocarbyl-substituted succinic acid or anhydride and hydrazine are reacted in a molar ratio of 2:1 - 1:4, preferably, 1:1 - 1:3.

5. A diesel fuel composition according to any preceding claim wherein the reaction product between the hydrocarbyl-substituted succinic acid or anhydride and hydrazine is added to the diesel fuel in an amount of between 20 and 100ppm based on the weight of the fuel.

## Patentansprüche

1. Dieselkraftstoffzusammensetzung, die eine größere Menge an Dieselkraftstoff und zwischen 10 und 500 Gew.-ppm, bezogen auf das Gewicht des Kraftstoffs, des Reaktionsprodukts zwischen einer mit Kohlenwasserstoff substituierten Bernsteinsäure oder einem mit Kohlenwasserstoff substituierten Bernsteinsäureanhydrid und Hydrazin umfasst, wobei mindestens 25 Gew.-%, vorzugsweise mindestens 50 Gew.-%, insbesondere mindestens 80 Gew.-% des Reaktionsprodukts ein Molekulargewicht aufweisen, das mehr als das zweifache, insbesondere mehr als das 2,5-flache des durchschnittlichen Molekulargewichts der Kohlenwasserstoffgruppe der mit Kohlenwasserstoff substituierten Bernsteinsäure oder des mit Kohlenwasserstoff substituierten Bernsteinsäureanhydrids beträgt.

2. Dieselkraftstoffzusammensetzung nach Anspruch 1, wobei das Reaktionsprodukt zwischen der mit Kohlenwasserstoff substituierten Bernsteinsäure oder dem mit Kohlenwasserstoff substituierten Bernsteinsäureanhydrid und Hydrazin vorwiegend eine oligomere Spezies mit der Struktur 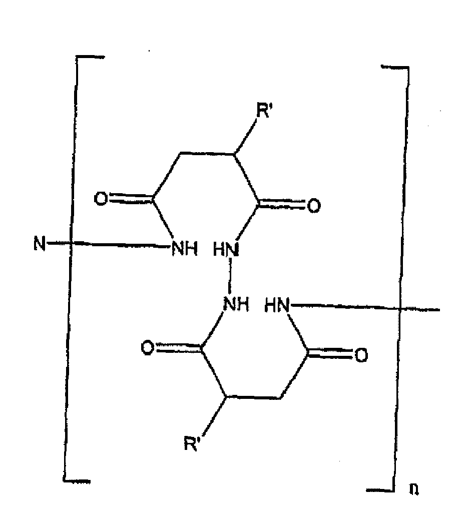 ist, in der R' den Kohlenwasserstoffsubstituenten wiedergibt und in der n eine ganze Zahl und größer als 1 ist, vorzugsweise zwischen 2 und 10, insbesondere zwischen 2 und 7, zum Beispiel 3, 4 oder 5.

3. Dieselkraftstoffzusammensetzung nach Anspruch 1 oder Anspruch 2, wobei die Kohlenwasserstoffgruppe der mit Kohlenwasserstoff substituierten Bernsteinsäure oder des mit Kohlenwasserstoff substituierten Bernsteinsäureanhydrids eine C₈ bis C₃₆-Gruppe oder eine Polyisobutylengruppe umfasst, die ein Molekulargewicht (Zahlenmittel) von zwischen 200 und 2500 aufweist.

4. Dieselkraftstoffzusammensetzung nach einem der vorhergehenden Ansprüche, wobei die mit Kohlenwasserstoff substituierte Bernsteinsäure oder das mit Kohlenwasserstoff substituierte Bernsteinsäureanhydrid und Hydrazin in einem molaren Verhältnis von 2:1 bis 1:4, vorzugsweise 1:1 bis 1:3 umgesetzt sind.

5. Dieselkraftstoffzusaznmensetzung gemäß einem der vorhergehenden Ansprüche, wobei das Reaktionsprodukt zwischen der mit Kohlenwasserstoff substituierten Bernsteinsäure oder des mit Kohlenwasserstoff substituierten Bernsteinsäureanhydrids und Hydrazin dem Dieselkraftstoff in einer Menge zwischen 20 und 100 ppm zugesetzt wird, bezogen auf das Gewicht des Treibstoffs.

## Revendications

1. Composition de carburant diesel comprenant une quantité dominante d'un carburant diesel et 10 à 500 ppm en poids, sur la base du poids du carburant, du produit de réaction entre un acide ou anhydride succinique à substituant hydrocarbyle et de l'hydrazine, dans laquelle une proportion d'au moins 25 %, avantageusement d'au moins 50 %, de préférence d'au moins 80 % en poids du produit de réaction, a un poids moléculaire qui est égal à plus de 2 fois, de préférence plus de 2,5 fois, le poids moléculaire moyen du groupe hydrocarbyle de l'acide ou de l'anhydride succinique à substituant hydrocarbyle.

2. Composition de carburant diesel suivant la revendication 1, dans laquelle le produit de réaction entre l'acide ou l'anhydride succinique à substituant hydrocarbyle et l'hydrazine est de manière prédominante une entité oligomère de structure : 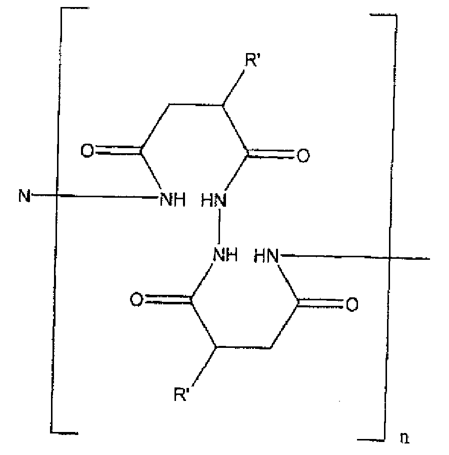 dans laquelle R' représente le substituant hydrocarbyle et dans laquelle n représente un nombre entier et est supérieur à 1, et a avantageusement une valeur de 2 à 10 , plus avantageusement de 2 à 7, par exemple est égal à 3, 4 ou 5.

3. Composition de carburant diesel suivant la revendication 1 ou la revendication 2, dans laquelle le groupe hydrocarbyle de l'acide ou de l'anhydride succinique à substituant hydrocarbyle comprend un groupe en C₈ à C₃₆ ou un groupe polyisobutylène ayant une moyenne en nombre du poids moléculaire de 200 à 2500.

4. Composition de carburant diesel suivant l'une quelconque des revendications précédentes, dans laquelle l'acide ou l'anhydride succinique à substituant hydrocarbyle et l'hydrazine sont amenés à réagir en un rapport molaire de 2:1 à 1:4, de préférence de 1:1 à 1:3.

5. Composition de carburant diesel suivant l'une quelconque des revendications précédentes, dans laquelle le produit de réaction entre l'acide ou l'anhydride succinique à substituant hydrocarbyle et l'hydrazine est ajouté au carburant diesel en une quantité de 20 à 100 ppm sur la base du poids du carburant.
